(19) Europäisches Patentamt
European Patent Office
Office européen des brevets

(11) **EP 4 308 075 B1**

(12) **EUROPEAN PATENT SPECIFICATION**

(45) Date of publication and mention
of the grant of the patent:
**29.04.2026 Bulletin 2026/18**

(21) Application number: **22712660.4**

(22) Date of filing: **15.03.2022**

(51) International Patent Classification (IPC):
***A61K 9/00*** *(2006.01)* ***A61K 47/30*** *(2006.01)*

(52) Cooperative Patent Classification (CPC):
**A61K 9/0021; A61K 9/0065; A61K 47/30**

(86) International application number:
**PCT/IL2022/050290**

(87) International publication number:
**WO 2022/195586 (22.09.2022 Gazette 2022/38)**

(54) **EXPANDABLE DEVICES FOR DELIVERY OF ACTIVE AGENTS TO TISSUES**

EXPANDIERBARE VORRICHTUNGEN ZUR ABGABE VON WIRKSTOFFEN AN GEWEBE

DISPOSITIFS EXPANSIBLES POUR L'ADMINISTRATION D'AGENTS ACTIFS À DES TISSUS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **15.03.2021 US 202163161207 P**
**02.08.2021 US 202163228409 P**

(43) Date of publication of application:
**24.01.2024 Bulletin 2024/04**

(73) Proprietor: **Epitomee Medical Ltd.**
**Caesarea 3079892 (IL)**

(72) Inventors:
• **COHEN, Gil**
9782224 Jerusalem (IL)
• **ARTAMONOV, Valery**
7751019 Ashdod (IL)
• **HASHIMSHONY, Dan**
3713227 Pardes Hana (IL)

(74) Representative: **Plasseraud IP**
**104 Rue de Richelieu**
**CS92104**
**75080 Paris Cedex 02 (FR)**

(56) References cited:
WO-A1-2015/083171 WO-A2-2009/125432
US-A1- 2015 150 701

## Description

### TECHNOLOGICAL FIELD

This disclosure concerns expandable devices, specifically self-deployed devices, for adherence to a tissue, *e.g.* intestinal tissue, and, for delivery of at least one active agent to, or across, a tissue.

### BACKGROUND ART

References considered to be relevant as background to the presently disclosed subject matter are listed below:

- PCT patent publication WO2016015648
- PCT patent publication WO2008062440
- PCT patent publication WO2009125432
- PCT patent publication WO2013188819
- PCT patent publication WO2015026552
- PCT patent publication WO2015120471

Acknowledgement of the above references herein is not to be inferred as meaning that these are in any way relevant to the patentability of the presently disclosed subject matter.

### BACKGROUND

Effective and targeted delivery of various compounds and active agents to or across a tissue, for example the lining the intestinal tract, has proven over the years to be a challenge. Due to mucus secretions that line various tissues for providing a hydrated environment and lubrication of biological surfaces, targeted delivery of active agents often requires the use of mucoadhesive components or other tissue attachment arrangements, which are capable of attaching to the tissue for a pre-defined period of time, thereby providing longer contact time between the tissue and the active agent to allow longer time of absorption of the active agent through and/or to the tissue.

While many of the approaches utilized often involve compositions that comprise mucoadhesive components in particulate form (*e.g.* capsules that comprise an active agent dispersed in mucoadhesive particles, or devices that disperse the mucoadhesive particles when exposed to defined conditions), such are difficult to disperse once in vicinity of the relevant tissue, and do not provide sufficient control over the location in which the mucoadhesive is adhered to the tissue. Further, such systems often suffer from insufficient adhesion and/or insufficient residence time in the target site.

Other approaches have suggested utilizing various applicators to bring the mucoadhesive material into contact with the target tissue. Utilizing such applicators is often of invasive nature, and hence less suitable for delivery of active agents into internal organs (such as the gastrointestinal tract).

### GENERAL DESCRIPTION

The present disclosure provides self-expandable devices, which can be administered to a subject and undergo a controlled deployment or adhesion of one or more tissue-attachable layers (or patches), *e.g.* mucoadhesive materials, to a tissue. This tissue-attachable layer contains one or more active agents which can be delivered to, and/or across, the tissue to which the tissue-attachable layer has been attached.

Additionally, being able to achieve good adhesion and sufficient adhesion time of a mucoadhesive layer can be useful for blocking transfer of ions, molecules, and/or small particles across the tissue.

The devices of this disclosure are designed to controllably increase in volume, such that volume increase drives a tissue-attachable layer or patch, *e.g.* a layer of mucoadhesive material, towards the tissue and temporarily applies force thereonto in order to attach or adhere the layer or patch to the tissue at a desired location. Hence, once the device is administered, exposure to the proper conditions for deployment will cause the device to expand and deliver the tissue-attachable layer to the tissue.

Thus, in one aspect of the present disclosure, there is provided a self-expandable device comprising at least one self-expandable compartment formed out of a substantially continuous, deformable film having one or more liquid-permeable sections, the compartment enclosing a gel forming material therein, the gel forming material being configured for swelling upon contact with liquid; and a tissue-attachable layer coating at least a portion of the external surface of the compartment, the device configured for attaching a tissue-attachable layer to a tissue by irreversible expansion of the device from a collapsed state to an expanded state upon contact of the gel forming material with liquid.

By another aspect, the disclosure provides a self-expandable device configured for attaching a tissue-attachable layer to a tissue, the device having a collapsed state and an expanded state. The device comprises at least one self-expandable compartment formed out of a substantially continuous, deformable film having one or more liquid-permeable sections, the compartment enclosing a gel forming material therein, that is configured for swelling upon contact with liquid, thereby expanding the compartment to irreversibly switch the device from the collapsed state to the expanded state. The device also comprises a tissue-attachable layer that coats at least a portion of the external surface of the compartment, such that expansion of the device from the collapsed state to the expanded state causes expansion of the compartment to drive the tissue-attachable layer towards said tissue for attaching at least part of the tissue-attachable layer to the tissue.

By a further aspect, this disclosure provides a self-expandable device for delivery of at least one active agent to, and/or across, a tissue, the device having a collapsed state and an expanded state. The device comprises at least one self-expandable compartment formed out of a substantially continuous, deformable film having one or more liquid-permeable sections, the compartment enclosing a gel forming material therein, that is configured for swelling upon contact with liquid, thereby expanding the compartment to irreversibly switch the device from the collapsed state to the expanded state. The device also comprises a tissue-attachable layer coating at least a portion of an external surface of the compartment, the tissue-attachable layer being composed of at least one mucoadhesive material and at least one active agent. Expansion of the device from the collapsed state to the expanded state causes expansion of the compartment, thus driving the tissue-attachable layer towards the tissue for adhering at least part of the tissue-attachable layer to the tissue to permit delivery of the active agent to, and/or across, the tissue.

In other words, the device comprises a closed compartment formed out of a deformable film that has one or more liquid-permeable sections, and holds therein one or more gel forming materials. For administration, the device is in a collapsed state, *i.e.* having a compact configuration with a given initial volume. Once administered and exposed to the proper conditions, as will be further discussed below, penetration of liquid through the liquid-permeable sections of the film causes the gel forming material to swell, thereby increasing in volume and irreversibly deploying the device into an expanded state. The outer surface of the compartment is at least partially coated by a tissue-attachable layer, for example a mucoadhesive layer. Hence, expansion of the compartment into the expanded state drives and/or pushes the tissue-attachable layer towards the tissue and brings it into contact with the tissue to permit attaching or adhering thereto.

In another aspect, there is provided a self-expandable device configured for attaching a tissue-attachable layer to a tissue, the device having a non-expanded (collapsed) state and an expanded state, and comprising: at least one self-expandable core formed of a substantially continuous, closed deformable film having one or more liquid-permeable sections and having a collapsed configuration in the non-expanded state of the device, the film enveloping a volume holding at least one gel forming material that is configured to expand upon contact with liquid, thereby increasing the volume of the core and deploying the film to a deployed configuration to irreversibly switch the device from the non-expanded state to the expanded state; and a tissue-attachable layer coating at least a portion of the outer surface of the core, such that expansion of the device from the non-expanded (collapsed) state to the expanded state, causes deployment of the core to drive the tissue-attachable layer towards said tissue for attaching at least part of the tissue-attachable layer to the tissue.

The *tissue-attachable layer* is meant to denote a layer which is capable of self-attaching to a tissue, typically a mucosal epithelial tissue. The attachment of the tissue-attachable layer to the tissue can be by mechanical means, *e.g.* microneedles or microhooks, which can temporarily anchor into the tissue upon application of pressure by the self-expandable core. Alternatively, the tissue-attachable layer can be a mucoadhesive layer.

The tissue-attachable layer comprises at least one active agent to be delivered to the tissue. For example, a mucoadhesive layer can comprise at least one mucoadhesive material and at least one active agent, and in such embodiments, attachment of the mucoadhesive layer to the tissue permits delivery of said active agent to, or across, the tissue. In another example, the microneedles are comprised of a polymeric material in which at least one active agent is embedded.

In the context of the present disclosure, the term *tissue* refers to any organ surface or biological membrane that may typically be coated by mucus or mucous membrane (e.g. the mucosal epithelial tissue). For example, the tissue can be gastric tissue, intestinal tissue, rectal tissue, vaginal tissue, urine tract tissue, nasal tissue, *etc.*

The term *mucoadhesive* (or any lingual variation thereof) is meant to denote a compound or composition of matter that is capable of adhering to a tissue, typically via the mucus or mucous membrane. The mucoadhesive material typically interacts with mucus present on or secreted by the tissue, *e.g.* one or more interactions such as electrostatic interaction, physical entanglement or interpenetration, diffusion, adsorption, mechanical interlocking, *etc.*

The mucoadhesive material is typically a polymer, that can be natural, semisynthetic or synthetic. Non-limiting examples of mucoadhesives are tragacanth, sodium alginate, guar gum, xanthan gum, karaya gum, gellan gum, carrageenan, soluble starches, gelatin, chitosan, cellulose derivatives (such as methylcellulose, ethylcellulose, hydroxylethylcellulose, hydroxylpropylcellulose, hydroxypropylmethylcellulose, sodium carboxymethylcellulose (NaCMC)), polyacrylic acid (PAA) polymers (such as carbomers, polycarbophil), polyhydroxyl ethylmethylacrylate, polyethyleneoxide (PEO, typically high molecular weight PEO), polyvinylpyrrolidone (PVP), polyvinyl alcohol (PVA), lectins, pectin, thiolated polymers (for example chitosan-iminothiolane), poly(acrylic acid)-cysteine, poly(acrylic acid)-homocysteine, polyethylene glycol, chitosan-thioglycolic acid, chitosan-thioethylamidine, alginate-cysteine, poly(methacrylic acid)-cysteine, sodium carboxymethylcellulose-cysteine, and others.

In some embodiments, the mucoadhesive layer may further comprise one or more additional components. Such additional components may be, for example, emulsifying agents (surfactants), such as poloxamers or carbomers; stabilizing agents, such as carboxymethylcellulose; suspending agents, such as cellulose, talc; acidifying agents, such as citric acid, ascorbic acid; viscosity increasing agents, such as carbopol, polyethylene oxide; effervescent agents, such as sodium bicarbonate, ammonium carbonate; solubilizing agents, such as lecithin; antimicrobial preservatives, such as sorbic acid, potassium sorbate; antioxidants, such as alpha tocopherol, butylhydroxy anisole; release modifying agents, such as Tween 80, sodium lauryl sulfate; coating agents, such as ethylcellulose, cellulose acetate; binders, such as hydroxypropyl cellulose, polyvinylpyrrolidone; stiffening agents, such as stearic acid, wax; plasticizers, such as diethyl phthalate, triethyl citrate; and others.

Depending on the target site and the desired contact time between the mucoadhesive layer and the tissue, the mucoadhesive can be selected to provide longer (stronger mucoadhesion) or shorter (weaker mucoadhesion) residence time on the tissue. The mucoadhesion strength can be influenced, *inter alia*, by the molecular weight of the polymer, degree of cross-linking, chain length and flexibility, electrical charges and polarity, pH, concentration of the mucoadhesive composition, *etc.*

The tissue-attachable layer, *e.g.* mucoadhesive layer, at least partially coats the external surface of the compartment, *i.e.* the tissue-attachable layer can coat one or more regions of the surface, a plurality of spaced-apart surface regions, or can be a continuous coating of the surface. In other embodiments, the tissue-attachable layer substantially coats the entire external surface of the compartment.

In yet other embodiments, in the collapsed state, the tissue-attachable layer is a sheet of mucoadhesive material having a non-extended (*e.g.* folded or rolled) configuration, with a portion of the tissue-attachable layer coating at least a region of the external surface of the compartment, and having an extended configuration in the expanded state in which the sheet extends (*e.g.* unfolds or unrolls) beyond the compartment.

In other words, the tissue-attachable layer can be formed out of a sheet of mucoadhesive material having one or more dimensions (*i.e.* length and/or width) that are larger than those of the compartment. In order to permit administration, the sheet is folded and/or rolled into a non-extended configuration, such that a portion of the sheet coats at least a region of the compartment. Once administered, concomitant with the expansion of the compartment, the sheet of mucoadhesive material can undergo unfolding and/or unrolling to assume an extended configuration, such that the extended sheet extends beyond the dimensions of the compartment. Such an arrangement enables delivery and deployment of tissue-attachable layers having dimensions larger than the compartment.

In some embodiments, when in the extended configuration of the expanded state, the tissue-attachable layer comprises a first portion coating at least a region of the external surface of the compartment, and a second portion extending beyond the compartment, the first and second portions being integral one with the other to form a mucoadhesive material sheet. In order to provide mechanical support to the second position of the mucoadhesive material sheet, the tissue-attachable layer may, by some embodiments, comprise a backing support layer over said second portion. In such embodiments, the tissue-attachable layer can comprises said backing support layer over one or both of the tissue-facing surface and the opposite, non-tissue facing surface of said second portion. The backing support layer can be made out of any suitable material, e.g. a non-mucoadhesive material that can be configured to disintegrate to permit exposure of the tissue-attachable layer during or after extension of the tissue-attachable layer and/or expansion of the device. When said backing support layer covers both of the tissue-facing surface and the non-tissue facing surface of said second portion, the type of layer may be different for the tissue-facing surface and the non-tissue facing surface.

Typically, the tissue is of a generally luminal organ or a hollow-cavity organ, such as the gastrointestinal tract (*e.g.* esophagus, stomach, small intestine, large intestine), sinuses, nasal cavities, vagina, uterus, fallopian tubes, urinary tract, rectum, *etc.*

As noted, the compartment is made of a continuous, deformable film. The term *continuous deformable film* means to denote a monolithic film, a film constructed as one seamless film, as well as a film that is constructed out of film segments that are welded together to form a continuous structure. The continuous, deformable film has one or more liquid-permeable sections to permit penetration of liquid into the compartment and expansion of the gel forming material. Other sections of the deformable film may be non-permeable to liquids; hence, in some embodiments, the deformable film, save said or more sections of liquid-permeable material, can be made of non-permeable material. In other words, the deformable film can be made out of two or more different materials, integrally formed one with the other, one material being liquid-permeable and the other material being non-permeable to liquid.

By some embodiments, said one or more sections of the deformable film differ one from the other in their liquid permeability. For example, the sections can differ in their composition, porosity, thickness, size and density of perforations, *etc.*

By other embodiments, the entire deformable film is made out of liquid-permeable material, *i.e.* a continuous film of liquid permeable material. In such embodiments, the deformable film can be made in its entirety out of a single liquid-permeable material, or out of two or more segments, integrally formed one with the other to form the deformable film, each of the segments being made of a different liquid permeable material.

In some embodiments, the deformable film and/or the gel forming material are disintegrable. Hence, after expansion and attachment of the tissue-attachable layer to the tissue, the compartment components are relatively quickly disintegrated (*e.g.* mechanically, physically, and/or chemically), and can be cleared from the deployment site, to leave the tissue-attachable layer attached to the tissue. In some embodiments, the deformable film comprises weak regions that are configured to permit fragmentation of the film after a pre-defined period of time from the expansion of the device. The weak regions can be generated, for example, by locally controlling the composition the film, the mechanical structure of the film, the construction the film (*e.g.* bonding and/or welding regions), *etc.*

Clearance of the tissue-attachable layer from the tissue typically occurs by shedding of tissue cells and detachment from the target site. For example, the intestinal tissue is shed every few hours, and hence a tissue-attachable layer that is attached thereto will clear from the target site together with the shed tissue. Hence, residence time of the tissue-attachable layer in the target site is typically, albeit not exclusively, determined by the nature of the tissue to which it is targeted to adhere. Alternatively, the residence time and/or detachment of the tissue-attachable layer from the tissue may be governed by the properties of the tissue-attachable layer (*i.e.* chemical or physical property).

In some embodiments, the deformable film and/or the gel forming material are chemically disintegrable. In other embodiments, the deformable film and/or the gel forming material are physically disintegrable. For example, the deformable film and/or the gel forming material may be tailored to disintegrate within 15 minutes to 3 hours, such as not to cause prolonged interference within the organ in which the device is deployed (*e.g.* the gastrointestinal tract).

In the expanded state, the device may have a circular shape, a polygonal shape, or an irregular shape. In its expanded state, the device may be designed to assume a three-dimensional (3D) shape generally conforming to the shape of at least a section of the lumen or cavity in which it expands (or deploys).

In other embodiments, in the expanded state, the device may have an annular or ring-like shape.

In some other embodiments, the device may be configured to assume a substantially cylindrical shape when in the expanded state, to define a hollow lumen. Such a hollowed cylindrical shape prevents formation of blockage of the luminal organ or organ cavity when the device is in its expanded state, permitting passage of liquids or solids through the organ while the device is deployed therein and attached to the tissue.

As the device is typically deployed in a luminal organ or an organ cavity, the device can assume an elongated form (for example a cylinder) when in the expanded state. In some embodiments, when in the expanded state, the device has a length to width (W/L) ratio of L/W of greater than about 1.5.

By some embodiments, the device is in the form of a sleeve, the sleeve walls being constituted by the deformable film, wherein the self-expandable compartments are defined along the circumference of the sleeve, with the tissue-attachable layers coating at least a portion of the external surface of the compartments and facing outwards from the surface of the sleeve. Typically, the compartments are elongated along a longitudinal axis of the sleeve and are arranged parallel to one another along the circumference of the sleeve.

By some embodiments, each of the elongated compartments are horizontally segmented in order to form two or more sub-compartments, longitudinally arranged along the longitudinal axis of the sleeve. By such embodiments, each sub-compartment may carry a tissue-attachable layer on its external surface. The horizontal segmentation may provide another degree of flexibility to the device at its collapsed form, enabling further folding the device along the horizontal segmentation line in order to reduce its volume for encapsulating into a biodegradable shell for intake, as will be further disclosed below.

While the device can have a single compartment, it is also envisaged that the device can comprise two or more compartments, similar or different. The various expanded shapes of the device described above may be generated by use of multiple compartments. For example, a hollow cylindrical shape may be created by using multiple slender compartments attached to each other along their longitudinal axis, forming a ring. Thus, by some embodiments, the device can comprise two or more compartments, identical to one another or different from one another (*e.g.* in any one of size, shape, active agent, type, size or shape of tissue-attachable layer, disintegration rate, *etc.*). According to some embodiments, the device can comprise one or more compartments carrying tissue-attachable layers, while other compartments of the device may be devoid of said tissue-attachable layer.

When the device comprises two or more compartments, all of the compartments can be formed out of the continuous deformable film, e.g. by forming separation regions (for example by welding) to define perimeters of the compartments. Alternatively, each compartment may be made of a continuous deformable film, and the compartments can be attached (*e.g.* welded) one to the other in order to form the device.

By varying the size, geometry, number, *etc.* of the compartments, as well as the type of gel forming material and/or tissue-attachable layer, different expansion rates, expanded shapes and/or targeted delivery can be obtained. Varying the size, number, geometry, *etc.* of the compartments can also be utilized in obtaining symmetrical or non-symmetrical expanded shape of the device. Varying the properties of the gel-forming material and/or the deformable film can also permit controlling the force applied by the device, in its expanded state, on the tissue for adhering the tissue-attachable layer thereto. Further, by folding the device to obtain the collapsed state and/or controlling the number and/or position of the liquid-permeable sections of the deformable film, control over the exposure of the gel-forming material to the liquid can be obtained, thus controlling the overall expansion rate of the device.

As the compartment is made out of a closed, continuous deformable film, it defines an enclosed volume, that holds the gel-forming material. In other words, the continuous film fully encloses the gel-forming material. The film is typically flexible and/or deformable, thereby enabling its folding in various manners in order to reduce the size of the device and obtain the collapsed state, such that the device can be easily administered to a patient in need thereof and into the target organ or organ cavity. In some embodiments, device is encapsulated in its collapsed state in a self-administrable capsule, to swallowed by the patient.

By some embodiments, when in the collapsed configuration, the distance between adjacent compartments is no more than about 10 mm, for example, between about 1 and 5 mm.

By some embodiments, when in the collapsed configuration, the width of the compartments is no more than about 25 mm, for example, between about 8 and 20 mm.

In some embodiments, when in the collapsed state, the device is folded in a primary folded configuration and is configured to undergo unfolding during transition from the collapsed state to the expanded state. In other words, the device may be folded to assume its collapsed state, having an overall reduced size or overall reduced volume. Once liquid permeates through the liquid-permeating film, the gel-forming material starts to swell and increase in volume. This, in turn, applies force onto the deformable film, and due to its flexibility and/or deformability, the film is unfolded to assume the device's expanded state.

By some embodiments, the device is folded at least once along a longitudinal axis, or an axis angled with respect to the longitudinal axis of the device (*e.g.* along a horizontal midline), in order to obtain the primary folded configuration. By other embodiments, the device is folded at least once along both its longitudinal axis and an axis angled with respect to the longitudinal axis of the device for obtaining the primary folded configuration.

When in the primary folded configuration, the folded device, by some embodiments, can be enveloped by an enteric envelope. According to some embodiments, the enteric envelop is formed out of an enteric film. According to such embodiments, the enteric film comprises or is composed of one or more enteric polymers. The term *enteric polymer(s)* means to denote polymeric material (*i.e.* a single polymer or a composition of polymers) that are configured to be disintegrated or solubilized by a liquid only at a defined pH range. For example, and preferably, the enteric polymers are stable (that is, maintain their physical and chemical structure) when exposed to acidic environments (for example in the stomach), and are solubilized by more alkaline liquids (such as those in the intestine).

According to some embodiments, the enteric envelope is configured to hold the device in its primary folded configuration, for example by forming the enteric envelope to have dimensions similar to those of the device when in its primary folded configuration. By some embodiments, the enteric envelope is tightly fit over the device in its primary folded configuration, substantially without having spaces formed between the enteric envelope and the device.

By some embodiments, the enteric film comprises one or more additives, *e.g.* film-forming compounds, plasticizers, stabilizers, fillers, *etc.*

The enteric envelope functions to maintain the device in its folded configuration after intake, until reaching proper conditions (for example, proper pH) within the GI tract that degrade the enteric envelope to permit exposure of the device and its unfolding and expansion.

By some embodiments, in addition to the device, the enteric envelope encapsulates at least one anti-buoyancy element having a density higher than about 1 g/ml, to prevent the device from floating over the surface of the liquid in the stomach and improve the delivery of the encapsulated device to the intestine. The anti-buoyancy element can, in some embodiments, be constituted by one or more regions of larger thickness of the enteric envelope. By other embodiments, the anti-buoyancy element can be one or more mass units attached to the enteric envelope or positioned within the space formed by the enteric envelope. By some other embodiments, the space defined by the enteric envelope is divided into a primary space, enveloping the folded device, and one or more auxiliary spaces, containing the anti-buoyancy elements.

In order to obtain further compactization in the collapsed state, the device, by some embodiments, may have a secondary, rolled configuration, whereby the folded device is further rolled about an axis thereof, and is configured to simultaneously undergo unrolling and unfolding during transition from the collapsed state to the expanded state.

According to some embodiments, when in its secondary folded configuration, the device can be enveloped in an enteric envelope. Alternatively, the device can be enveloped by a first enteric envelope when in its primary folded configuration, and by a second enteric envelope when in its secondary folded configuration. In other words, the device can be enveloped by a first enteric envelope after folding it into the primary folded configuration, then folded or rolled into the secondary folded configuration, and enveloped by the second enteric envelope to maintain the device in its secondary folded configuration.

According to other embodiments, the device may first be rolled and then folded in order to assume the collapsed state.

By some other embodiments, in the collapsed state, the device is rolled about an axis thereof, and is configured to undergo unrolling during transition from the collapsed state to the expanded state (*i.e.* without unfolding).

According to some embodiments, in order to prevent adherence of the external layer of the device to itself when the device is in its primary and/or secondary folded configurations, one or more separation layers are placed between one or more of the folds of the device. These one or more separation layers can be attached in one or more attachment locations to the device in order to maintain their position and function when the device is folded into its primary and/or secondary folded configurations. Alternatively, the separation layer(s) can be located in between the folds without anchoring it to the device (for example by placing such a layer over the device before folding, and then folding the device together with the separation layer to form the primary and/or secondary folded configuration).

As noted, the compartment is formed out of a deformable film that has one or more liquid-permeable sections. The sections (or in some embodiments the entire deformable film) are made of liquid-permeable material. Within the context of the present disclosure, the term *liquid-permeable material* is meant to denote a material (compound or composition of matter) that permits diffusion or passage of liquid therethrough. For example, the liquid permeable material may be perforated or porous. According to some embodiments, the liquid-permeable material may comprise one or more compounds selected from hypromellose phthalate, cellulose acetate phthalate, hypromellose acetate succinate, cellulose acetate, cellulose acetate butyrate, ethylcellulose, polymethylmethacrylate, polyethylacrylate, polyvinyl acrylate phthalate, polyvinyl acetate, shellac, carboxymethylethylcellulose (CMEC), and any combinations thereof.

The liquid-permeable material may, according to some embodiments, further comprise at least one binder, plasticizer, pore-former, emulsifier, film-former, and any combinations thereof.

In order to permit degradation of the compartment after expansion of the device, the liquid-permeable material is typically biodegradable, preferably enterically degradable.

The term *biodegradable* is meant to denote any type of decomposition of the device that is caused by exposure to suitable biological conditions after administration and expansion. The term encompasses mechanical breakdown, chemical or physical degradation, chemical or physical decomposition, or any other type of destruction of the integrity of the device during its passage through the gastrointestinal tract for expulsion from the body.

The term *gel-forming material* is meant to denote a compound or a composition that is capable of absorbing liquid(s), thereby forming a three-dimensional voluminous network of molecules. The gel-forming material may form a physical gel (*i.e.* a gel in which molecules are held in the network by physical forces) or a chemical gel (*i.e.* a gel in which molecules are chemically bonded one to the other to form the network structure). By some embodiments, the gel-forming material comprises one or more gel forming compounds. By other embodiments, the gel-forming material comprises one or more additives.

According to some embodiments, the gel-forming material comprises one or more polymers. According to other embodiments, the gel-forming material may be charged or neutral.

According to some other embodiments, the gel-forming material is cross-linked or is cross-linkable. Without wishing to be bound by theory, the molecular weight of the gel forming material and the degree of cross-linking have significant impact on the gel's consistency (for example, hardness or rigidity), as well as on its rheological properties (*e.g.* viscosity). Hence, various molecular weights and cross-linking degrees are some of the parameters that can be used to control the behavior of the zones, thereby controlling the rate of deployment and/or the expanded size of the device.

In some embodiments, the gel-forming material may be selected from gelatin, alginate, chitosan, dextran, collagen, hyaluronic-acid, polyglutamic-acid, elastin, calcium polycarbophil, acrylamides, styrene maleic anhydride, polyethylene oxide, polyacrylic-acid, polyethylene glycol, carboxy methyl cellulose, polyvinyl pyrrolidone, sodium polyacrylate, hydroxypropyl methylcellulose or any combination or composition thereof.

By some embodiments, the gel-forming material is a composition comprising at least one charged gel-forming compound and at least one compound having an opposite charge, constructing a PEC (Poly Electrolyte Complex) formation upon liquid adsorption. In some embodiments, said at least one charged gel-forming compound is selected from polyvinyl acetate diethyl amino acetate (AEA), poly-lysine, chitosan, polymethacrylate (Eudragit E), poly-arginine. In other embodiments, said opposite charged compound is selected from gelatin, hyaluronic-acid, sodium polyacrylate, heparin, polyacrylic acid (Carbomer), alginate, pectin, carboxymethylcellulose.

In some other embodiments, the gel-forming material is at least one super absorbent polymer (SAP). The term *super absorbent polymer* refers to a polymer (typically cross-linked) or a polymer composition, that can absorb and retain large quantities of liquids, such as water (or liquids containing water), relative to the dry mass of the polymer. Non-limiting examples of SAP are polyethylene glycol (PEG), polyglutamic acid (PGA), polyacrylamide, alginic-acid, dextran, polyacrylic acid, carboxymethylcellulose (CMC), pullulan, starch, and any combinations thereof.

In some other embodiments, the gel-forming material has a swelling ratio of about 10 to 100 times-fold (w/w) (under conditions of: gastrointestinal tract pH at 37°C for 1 hour).

The term *swelling ratio* denotes the expansion extent of the gel-forming material between the state prior to adsorbing liquid (*i.e.* dry or semi dry form) and after adsorbing the maximal possible amount of liquid. The swelling ratio is weight-based determined and calculated according to the following equation:

$$[(wet\ weight) - (dry\ weight)]/[(dry\ weight)].$$

The gel-forming material can be in the form of a gel film (*i.e.* a substantially continuous layer of gel). According to some embodiments, the gel-forming material is in the form of gel particles. By other embodiments, the gel forming particles are in the form of a gel film, the gel particles embedded within a matrix forming a film. In some embodiments, the gel forming particles in the gel film are arranged in substantially a monolayer of gel particles.

According to yet other embodiments, the gel-forming material can be in the form of a powder. In some embodiments when the gel-forming material is in the form of gel particles, the average diameter of the particles of the gel-forming material can range between about 100 μm and about 300 μm.

According to some additional embodiments, the compartment may enclose a gas-forming composition in addition to the gel forming material.

As noted above, the device can comprise more than one compartment. In such devices, each of the compartments may comprise a different gel-forming material. In some other embodiments, all the compartments can comprise the same gel-forming material.

As can be understood, the device is constructed out of structural layers, *i.e.* layers that form, or are, a part of the structural formation of the device. Such layers, for example, are the one or more layers constituting the continuous deformable film, the inner envelope enveloping the device in its primary folded state, etc. Further, the device comprises functional layers, *i.e.* layers that have a desired functionality and are integral with the structural layers, for example the tissue-attachable layer.

The device can also comprise one or more auxiliar functional layers, to permit various additional functionalities, such as controlled release layer, separation layers, non-mucoadhesive layers, *etc.* The one or more auxiliar functional layers can be continuous or can be spaced-apart layer segments. The one or more auxiliar functional layers can be integral with the structural and/or functional layers, can be partially attached to the structural and/or functional layers, or can be non-attached to the structural and/or functional layers. By some embodiments, at least one auxiliar functional layer is sandwiched between the tissue-attachable layer and the external surface of the compartment (for example, a backing layer for holding/maintaining the tissue-attachable layer's integrity). By some embodiments, the auxiliar functional layer serves as a separation layer.

It should be understood that any of the types of layers which comprise the device (e.g. structural layers, functional layers, auxiliar functional layers) may themselves be constructed from a multilayer structure or from sublayers integrally formed one with the other.

The device is typically designed for delivery of at least one active agent to a target site through and/or across the tissue. Thus, as noted, the tissue-attachable layer can comprise or carry one or more such active agents. Depending on the type of active agent to be delivered (*e.g.* polarity, hydrophobicity/hydrophilicity, size, *etc.*), the agent can be contained within or onto the surface (external surface that is tissue facing, and/or inward-facing surface that is gel forming material-facing surface) of tissue-attachable layer.

In some embodiments, when the tissue-attachable layer is a mucoadhesive layer, the active agent is embedded within the mucoadhesive material. For example, the active agent can be dispersed or dissolved in the mucoadhesive material.

In other embodiments, the active agent can be encapsulated within various microparticulate or nanoparticulate structures, such as liposomes, microparticles, microcapsules, nanoparticles, or nanocapsules, the structures being distributed within the mucoadhesive material.

By another embodiment, the active material coats at least a surface portion of the tissue-attachable layer; for example, the active material can coat at least one portion of one or both of a tissue-facing surface or a deformable film-facing surface of the tissue-attachable layer.

According to further embodiments, the active agent can be linked to the mucoadhesive material by one or more linker moieties, that are susceptible to defined biological conditions, as to permit release of the active agent therefrom once exposed to such conditions.

In some embodiments, when the tissue-attachable layer comprises microneedles, the active agent can be embedded within the microneedles, for example within a polymer from which the microneedles are construed.

The active agent is typically a pharmaceutical active agent. The term *pharmaceutical active agent* means to denote molecules, compounds or compositions that are safe and effective for pharmaceutical use in a subject, typically mammals, and that possess the desired biological activity. The active agent can be selected, for example, from antibiotics, proteins, peptides, polypeptides, lipids, nucleic acids, hormones, steroids, antibodies, vitamins, anti-inflammatories, antihistamines, antiemetics, analgesics, chemotherapic agents, prophylactic agents, clotting factors, radiopharmaceuticals, contrasting agents, electrolytes, nutraceuticals, small molecules (of a molecular weight of less than about 1,000 Da or less than about 500 Da), *etc.*

In other embodiments, the active agent be comprised of microorganisms (e.g. intestine-friendly bacteria) and/or viruses.

In other embodiments, the active agent can be a nutraceutical compound.

The active agent can be in the form of a salt, acid-addition salt, free base, hydrate, solvate, or prodrug.

The active agent can be suitable for administration to humans. In other embodiments, the active agent can be a veterinary active agent.

The active agent is typically present in the tissue-attachable layer in a therapeutically effective amount. The *effective amount* for purposes herein may be determined by such considerations as known in the art. The amount must be effective to achieve the desired therapeutic effect, depending, *inter alia*, on the type and severity of the disease to be treated and the treatment regime. The effective amount is typically determined in appropriately designed clinical trials (dose range studies) and the person versed in the art will know how to properly conduct such trials in order to determine the effective amount. As generally known, the effective amount depends on a variety of factors including the affinity of the ligand to the receptor, its distribution profile within the body, a variety of pharmacological parameters such as half-life in the body, on undesired side effects, if any, on factors such as age and gender, and others.

The pharmaceutical active agent can be selected to induce at least one effect, *e.g. therapeutic effect*, which is capable of inducing, enhancing, arresting or diminishing at least one effect, by way of treatment or prevention of unwanted conditions or diseases in a subject. The at least one agent (substance, molecule, element, compound, entity, or a combination thereof) may be selected amongst therapeutic agents, *i.e.* agents capable of inducing or modulating a therapeutic effect when administered in a therapeutically effective amount.

In other embodiments, the active agent can be a diagnostic agent, *i.e.* an agent that permits diagnosis of one or more conditions or disorders. A *diagnostically effective amount* refers to an amount of the active agent, radiopharmaceutical or diagnostic composition, which allows for efficient molecular imaging depending on the type of the imaging technique (*e.g.* PET, SPECT, *etc.*) used, the acquisition parameters of the specific imaging technique used, the area of the body scanned, the physical condition of the subject, the purpose of the test or any other factors which are apparent to the person skilled in art.

In some embodiments, the device can comprise at least one additional active substance, being different from said at least one active agent. The additional active substance can have similar pharmaceutical activity as the active agent, or have a different pharmaceutical activity to that of the active agent.

By some embodiments, the mucoadhesive layer comprises the additional active substance. In such embodiments, the active agent and the additional active substance can have a co-therapeutic effect, *i.e.* additional or synergistic. For example, the additional active substance can function to increase permeation or bioavailability of the active agent, or can increase or enhance the therapeutic effect or bioactivity of the active agent.

By other embodiments, the additional active substance can be contained within the compartment, *e.g.* associated with the deformable film, associated with the gel-forming material, or mixed (or dispersed) into the gel-forming material. In such cases, the additional active substance may be selected to have an immediate or a short-term therapeutic effect, while the active agent can be selected to have a prolonged or sustained therapeutic effect.

In other embodiments, the active agent and the additional active substance can be selected from agents having similar or identical therapeutic effects. For example, the active agent and the additional active substance can be the same, however one being contained within the tissue-attachable layer and the other within the compartment, respectively. Such an arrangement can be utilized to divide the required dose of the agent, such that a portion of the agent is released immediately upon expansion and/or disintegration of the gel-forming material, and the rest will be slowly absorbed from the tissue-attachable layer once attached to the tissue. By another example, the active agent and the additional active substance can have similar effects, with the additional active substance having an immediate effect and the active agent having a prolonged or sustained effect.

In other words, the device can be used to *simultaneously* administer two or more pharmaceutically active agents, *i.e.* administered concurrently one after the other. Simultaneous administration may permit one agent in the combination to be administered within a certain time period (*e.g.* 5 minutes, 10 minutes or even a few hours) after the other, provided that the circulatory half-life concentration of the first administered agent in a combination is concurrently present in therapeutically effective amounts with the other agent administered thereafter. The time delay between administration of the agents may vary depending on the exact nature of the agents, the interaction between the individual agents, their respective half-lives, and on such other factors as easily recognized by the versed artesian.

In a further example, the active agent and the additional substance can have different effects, and the device is designed for *sequential* administration; meaning that a time difference exists between administering one agent and the other. Such time different may be short or may be significant, *i.e.* the first administered agent may no longer be present (or is present in subclinical amounts) in the bloodstream in a therapeutically effective amount when the second (or subsequent) agent is administered.

In some additional embodiments, the device may include more than one type of a tissue-attachable layer. Different regions of the external surface of the compartment and/or different compartments, may be coated by different types of tissue-attachable layers. These different types of tissue-attachable layers may include different types of active agents.

In some additional embodiments, the tissue-facing surface of the tissue-attachable layer (or portions thereof) may be covered by a non-mucoadhesive material (layer) that can be configured to disintegrate to permit exposure of the mucoadhesive layer during or after extension of the tissue-attachable layer and/or expansion of the device.

In order to prevent undesired or pre-mature deployment of the device, and/or in order to permit delivery of the device into the proper organ lumen or cavity, the device may comprise a biodegradable shell, encapsulating the device in its collapsed state. The biodegradable shell is thus selected to be degraded upon exposure to the proper biological conditions (*e.g.* pH, presence of certain chemical compounds, *etc.*).

When the device is intended for oral administration and designed to be deployed in the intestine, the biodegradable shell can be made of or coated by an enteric coating.

In some embodiments, the device is an ingestible device, intended, for example, to be deployed in the stomach or in the intestine. Thus, such devices are typically encapsulated within a shell degradable in the gastrointestinal track, encapsulating the device in its collapsed state. For deployment in the intestine, the shell can be designed or selected such as to provide safe first passage through the stomach and biodegrade only when exposed to defined conditions in the intestine. By some embodiments, the gastrointestinal track degradable shell is designed to disintegrate as a function of the pH of the environment, thereby deploying in the desired part of the intestine. For example, different parts of the gastrointestinal tract are known to have different pH values - while the stomach typically has a pH 1.5-3.5, the pH in the duodenum is typically 6, and gradually increasing to about 7.4 in the small intestine (until reaching the terminal ileum). The pH drops to 5.7 in the caecum, but again gradually increases, reaching pH 6.7 in the rectum. Hence, by utilizing a gastrointestinal track degradable shell that degrades at defined pH values (or range of values), deployment of the device at the desired part of the gastrointestinal track can be obtained.

The biodegradable shell can be, for example, a capsule.

When the device is an ingestible device, the biodegradable capsule typically has a size that is suitable for swallowing, *e.g.* of about "elongated 000" or 000 or 00 capsule or less (*i.e.* outer diameter of about 9.97mm or less, height or locked length of about 30.0mm or less and actual volume of about 1.68ml or less). **Table 1** below provides non-limiting capsule sizes suitable for use as gastrointestinal track degradable shells.

**Table 1:** Degradable shell (capsule) sizes

| Capsule size | Outer Diameter (mm) | Height or Locked Length (mm) | Actual Volume (ml) |
|---|---|---|---|
| Elongated 000 | 9.97 | 30.0 | 1.68 |
| 000 | 9.97 | 26.14 | 1.37 |
| 00 | 8.53 | 23.30 | 0.95 |
| 0 | 7.65 | 21.70 | 0.68 |
| 1 | 6.91 | 19.40 | 0.50 |
| 2 | 6.35 | 18.00 | 0.37 |
| 3 | 5.82 | 15.90 | 0.30 |
| 4 | 5.31 | 14.30 | 0.21 |
| 5 | 4.91 | 11.10 | 0.13 |

When the device is an ingestible device, it may have two types of shells, one encasing the other. The outer shell in the form of a capsule, to facilitate the swallowing of the device, and is designed to be degradable in the gastric environment. The second, inner, shell may be a coating layer or an encapsulating layer, that coats/encapsulates the device, and is configured to maintain the device in its collapsed state for a predetermined period of time before deployment. For example, the inner shell can be made from an enteric material, thus preventing deployment of the device in the stomach and permitting its deployment only upon entrance to the intestine.

While the device can be used to deliver at least one active agent to a target site, the device can also be devoid of active agents, or comprise only the active substances within the compartment. For example, the device can be utilized to deliver a patch to a wall of a tissue, *e.g.* to temporarily block passage of substances across the mucosal/epithelial tissue (both from the lumen and/or to the lumen), or to cover perforations or ulcers in the tissue.

As noted, in some embodiments, while the tissue-attachable layer may not contain an active agent, the compartment and/or the gel-forming material may comprise an active substance/agent, such as an analgesic or anti-inflammatory, that can be released during the disintegration of the gel-forming material to provide a local desired effect relatively quickly after administration.

According to another aspect, the present disclosure provides a self-expandable device configured for delivery of at least one active agent to a tissue, the device having a collapsed state and an expanded state and encapsulated in a degradable shell when in the collapsed state, the device comprising: at least one self-expandable compartment formed out of a substantially continuous, deformable film having one or more liquid-permeable sections, the compartment enclosing a gel forming material therein, the gel forming material being configured for swelling upon contact with liquid, thereby expanding the compartment to irreversibly switch the device from the collapsed state to the expanded state; and a tissue-attachable layer coating at least a portion of the external surface of the compartment, the tissue-attachable layer comprising at least one mucoadhesive material and at least one active agent, such that expansion of the device from the collapsed state to the expanded state causes expansion of the compartment to drive the tissue-attachable layer towards said tissue for adhering at least part of the mucoadhesive layer to the tissue for delivery of said active agent to the tissue.

According to a further aspect, there is provided a self-expandable device configured for attaching a tissue-attachable layer to a tissue, the device having a collapsed state and an expanded state and encapsulated in a degradable shell when in the collapsed state, the device comprising: at least one self-expandable compartment formed out of a substantially continuous, deformable film having one or more liquid-permeable sections, the compartment enclosing a gel forming material therein, the gel forming material being configured for swelling upon contact with liquid, thereby expanding the compartment to irreversibly switch the device from the collapsed state to the expanded state; and a tissue-attachable layer coating at least a portion of the external surface of the compartment, such that expansion of the device from the collapsed state to the expanded state causes expansion of the compartment to drive the tissue-attachable layer towards said tissue for adhering at least part of the mucoadhesive layer to the tissue.

According to yet another aspect, there is provided a self-expandable device configured for attaching a tissue-attachable layer to a tissue, the device having a collapsed state and an expanded state, and comprising: a substantially continuous, deformable film formed into a sleeve, and defining at least one self-expandable compartment, said compartment having one or more liquid-permeable sections and enclosing a gel forming material therein, the gel forming material being configured for swelling upon contact with liquid, thereby expanding the compartment to irreversibly switch the device from the collapsed state to the expanded state; and a tissue-attachable layer coating at least a portion of the external surface of said compartment, such that expansion of the device from the collapsed state to the expanded state causes expansion of the compartment to drive the tissue-attachable layer towards said tissue for adhering at least part of the mucoadhesive layer to the tissue.

According to another aspect, there is provided a kit comprising a self-expandable device as described herein, encapsulated in a biodegradable shell, and instructions for use.

By another one of its aspects, the disclosure provides a method for delivery of at least one active agent to a tissue of a subject in need thereof, the method comprising administering to the subject a self-expandable device as disclosed herein, encapsulated in a biodegradable shell.

By a further aspect, there is provided a method of prolonged or sustained delivery of at least one active agent to a tissue of a subject in need thereof, the method comprising administering to the subject a self-expandable device as disclosed herein, encapsulated in a biodegradable shell. Hence, the devices of this disclosure form a reservoir of active agent to be delivered at the target site for a prolonged period of time.

By yet a further aspect, there is provided a method of attaching, through a mucous membrane, a tissue-attachable layer to a tissue of a subject in need thereof, the method comprising administering to the subject a self-expandable device as disclosed herein, encapsulated in a biodegradable shell.

## BRIEF DESCRIPTION OF THE DRAWINGS

In order to better understand the subject matter that is disclosed herein and to exemplify how it may be carried out in practice, embodiments will now be described, by way of non-limiting example only, with reference to the accompanying drawings, in which:

**Figs. 1A-1G** are schematic representations of a device according to several exemplary embodiments of this disclosure;
**Figs. 2A-2F** are schematic representations of a sequence of operation of the device after administration to a subject;
**Fig. 2G** is a schematic representation of a non-symmetrical device according to an embodiment of this disclosure;
**Fig. 2H** is a schematic representation of a device according to this disclosure, with non-symmetrical adherence of a tissue-attachable layer to the tissue;
**Figs. 3A-3D** are exemplary cross sections of the device in its expanded state;
**Figs. 3E-3F** are longitudinal cross-sections through devices of Figs. 3A and 3B, respectively;
**Figs. 3G-3H** show cross sections where the device is designed to conform to a lumen or cavity with a varying cross section;

**Fig. 3I** shows a cross section where the device comprises compartments of different size;

**Figs. 4A-4B** are exemplary cross sections of further configurations of the device in its expanded state;

**Figs. 5A-5B** are schematic representations of a device according to some further embodiments of this disclosure;

**Figs. 6A-6C** are schematic representations of a device according to additional embodiments of this disclosure, in which the device is configured as a sleeve: Fig. 6A being a front perspective view Fig. 6B being a top cross-sectional view across line V-V, Fig. 6C showing the device in an expanded form;

**Fig. 6D-6F** show various folding configurations of the device of Figs. 6A-6B;

**Figs. 6G-6H** shows the folded configuration of the device of Fig. 6E, enveloped in an enteric envelope - top view (Fig. 6G) and side view (Fig. 6H);

**Figs. 7A-7B** are schematic representations of some variations of the device of Figs. 6A-6B;

**Figs. 7C-7D** shows the device of Fig. 7A or 7B, in possible primary folded configurations;

**Figs. 7E-7F** show the devices of Figs. 7C-7D, respectively, covered by an enteric envelope.

## DETAILED DESCRIPTION OF EMBODIMENTS

In the following, exemplary devices according to this disclosure will be described. While the specific examples show the device as being substantially symmetric, it is to be understood that the device may also be asymmetric or of any other shape. Further, the elements of the device are shown out of scale for ease of illustration.

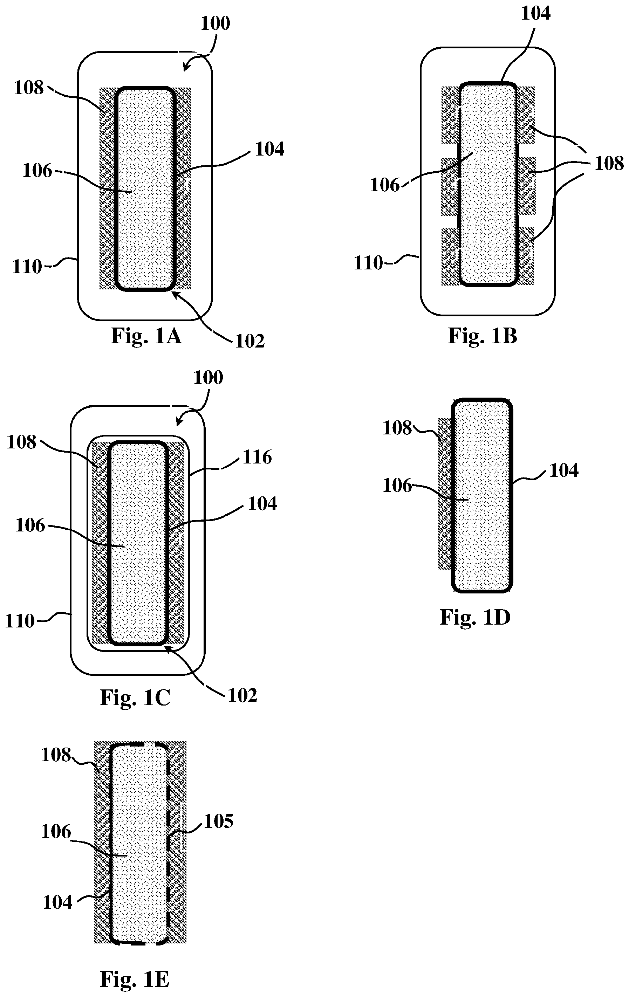

Shown in Fig. 1A is a self-expandable device according to an embodiment of this disclosure, in a collapsed (non-expanded) state. The device **100** includes a self-expandable compartment, generally designated **102,** that is formed out of a continuous deformable film **104.** In this specific example, the entire deformable film is made of liquid-permeable material; however, as noted herein it is to be understood that the deformable film may have one or more sections that are liquid-permeable (not shown), and other sections which are not liquid-permeable. At least part of the volume of the compartment is filled with gel-forming material **106.** Tissue-attachable layer **108** of this embodiment, *e.g.* a mucoadhesive layer or a layer comprising microneedles or microhooks, substantially coats the external surface of the compartment **102** (*i.e.* the face of the film **104** that faces outwards from the compartment). When the device is intended for delivery of one or more active agents to the tissue, the tissue-attachable layer **108** comprises the active agent(s). In order to permit administration, for example by oral intake for deployment in the intestine, the device is encapsulated by a biodegradable (e.g. gastric degradable) capsule **110,** that enables the device to pass the stomach intact and undeployed.

Another embodiment of the device is shown in Fig. 1B, in which the tissue-attachable layer coats spaces-apart zones of the external surface of the compartment.

The device can further comprise one or more additional active substances, *e.g.* contained within the compartment, for example intermixes with the gel-forming material **106.**

In the embodiment of Fig. 1C, another device is shown for oral intake, and is designed to deliver the tissue-attachable layer to the intestine. In this example, the device **100** is shown in a collapsed and encapsulated state. In addition to capsule **110,** which is designed to facilitate the swallowing of the device and intended for disintegration in the stomach, a second, inner shell **116** is an enteric envelope, that coats or encapsulates the device. The enteric envelop is configured to maintain the device in its collapsed state as long as the device is in the stomach, and disintegrates upon exposure to proper conditions in the intestine, thus enabling deployment of the device in the intestine and not in the stomach.

In another example, shown in Fig. 1D, the mucoadhesive layer **108** partially, and non-symmetrically coats a region of the compartment.

By another example, the compartment may be formed out of at least two different deformable films, **104** and **105** as seen in Fig. 1E, to cause asymmetrical deployment of the tissue-attachable layer. For example, film **104** can be made of a liquid-permeable material and film **105** may be made of non-liquid permeable film. Alternatively, films **104** and **105** can differ in their degree of liquid permeability.

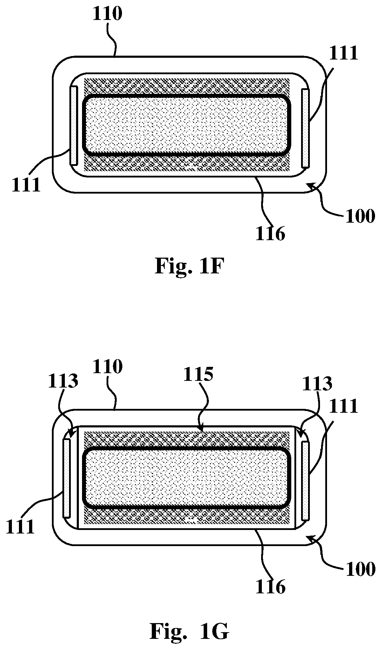

In another embodiment, shown in Figs. 1F-1G, the device is shown in a collapsed and encapsulated state, and the capsule **110** further contains anti-buoyancy elements **111** located within the enteric envelope **116.** Elements **111** typically have a density higher that the density of stomach liquid, *e.g.* higher than about 1 g/ml, to ensure submerging of the capsule in the stomach liquid and delivery of the device to the intestine.

In the example of Fig. 1F, elements **111** are attached to an inner surface of the enteric envelope. Alternatively, elements **111** can be constituted by regions of the enteric envelope having a larger thickness than the other regions of the enteric envelope.

In the example of Fig. 1G, enteric envelope **116** defines an internal space, which is divided into main space **115** in which the folded device **102** is enveloped, and two auxiliary spaces **113** housing the anti-buoyancy elements **111.**

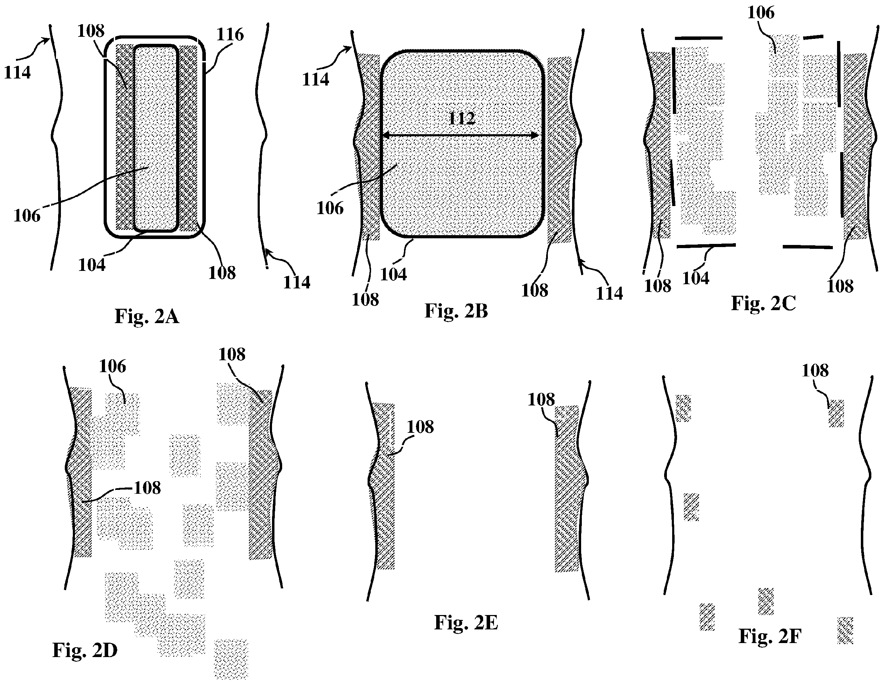

Shown in Figs. 2A-2E, is an exemplary sequence of operation of the devices of this disclosure. While this example shows deployment of the device in the intestine, it is to be understood that the device can be administered and deployed in any other suitable bodily lumen or cavity. For example, the device can be administered to other organs, such as urine tract, vagina, rectally, intranasally, *etc.* Where the target organ or cavity is relatively user-accessible, the device can be administered by utilizing a dedicated applicator (not shown), in order to insert the device into the organ or cavity.

After the device is administered, *e.g.* orally, the biodegradable capsule **110** passes the stomach and undergoes disintegration when exposed to suitable conditions in the intestine, thus exposing device **100.** As noted, in some configurations, as shown in Fig. 2A, the biodegradable capsule **110** is degraded in the stomach and the device is encapsulated/coated with an enteric layer (coating) **116,** that protects the device when passing along the stomach, and is designed to disintegrate when exposed to suitable conditions in the intestine. It is to be noted, however, that in other configurations, the layer **116** may be absent. The enteric layer typically comprises or is formed out of one or more enteric polymers.

Once the capsule **110** (or the enteric layer **116,** as shown in fig 2A) is disintegrated, liquid in the intestine permeates the liquid-permeable sections of deformable film **104** of compartment **102,** causing expansion of gel-forming material **106** contained therein. Expansion of the gel-forming material causes expansion of the compartment and deformation of film, as shown in Fig. 2B, causing the device to assume its expanded state. In the expanded state, the expansion of the gel-forming material causes application of force onto film **104,** in the direction of arrow **112,** forcing the tissue-attachable layer **108** to come into contact with the tissue **114** and adhering thereto. Hence, the transition of the device from its collapsed state to its expanded state drives the mucoadhesive layer associated with the compartment towards the tissue and brings it into intimate contact therewith under application of force (applied by the expanding gel-forming material).

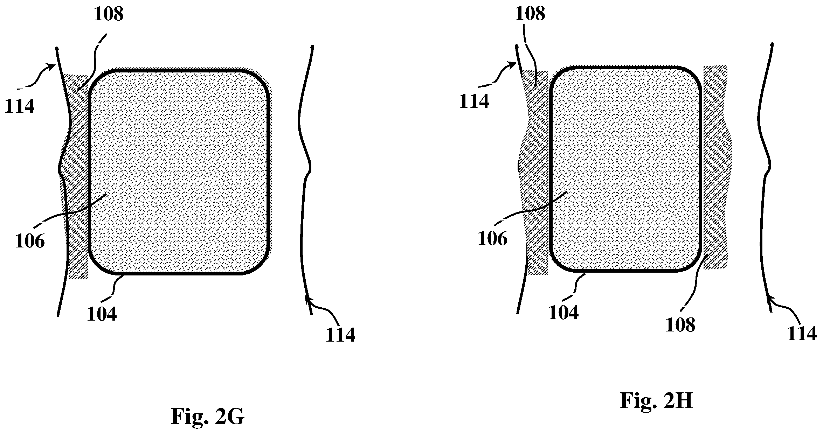

As seen in Figs. 2G and 2H, the mucoadhesive layer **108** does not need to symmetrically coat the deformable film and/or can adhere to the tissue in a non-symmetrical manner.

As shown in Figs. 2C-2D, the deformable film **104** and/or the gel forming material **106** undergo disintegration and are cleared from the target site by the intestinal natural movement, leaving the tissue-attachable layer attached or adhered to the tissue. When the tissue-attachable layer **108** contains an active agent, the active agent contained within the mucoadhesive layer is thus delivered to the tissue during the time period in which the tissue-attachable layer remains adhered to the tissue, as seen in Fig. 2E.

As the tissue is routinely shed from the intestinal wall every few hours, shedding of the tissue will result also in detachment and disintegration of the tissue-attachable layer, as seen in Fig. 2F, and clearance of the tissue-attachable layer from the intestine.

By some other embodiments, in the collapsed state, the device is rolled about an axis thereof, and is configured to undergo unrolling during transition from the collapsed state to the expanded state (*i.e.* without folding).

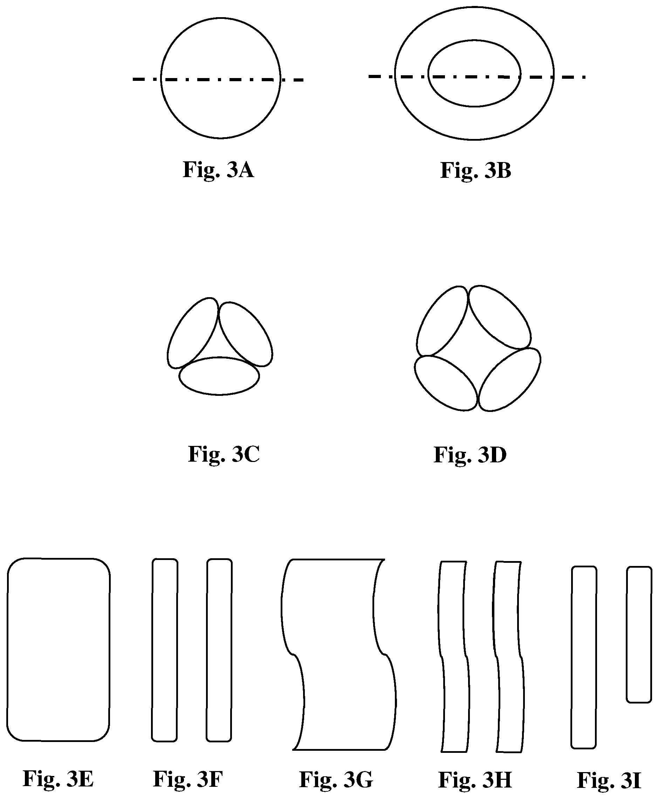

In the expanded state, the device may have a circular cross-section, a polygonal cross-section, or an irregular shape cross-section. Typically, in its expanded state, the device can assume a three-dimensional (3D) shape generally conforming to the shape of at least a section of the lumen or cavity in which it expands (is deployed). In specific exemplified embodiments, the compartment assumes a substantially cylindrical shape, with a circular cross-section as shown in Fig. 3A. However, it is also possible that the device assumes different shapes having different cross-sections when in the expanded state, such as that shown in Fig. 3B. As seen in Fig. 3B, the device can assume a cylindrical shape in its expanded state, with a hollow lumen. Such a hollowed cylindrical shape prevents formation of blockage of the organ cavity when the device is in its expanded state, permitting passage of liquids or solids through the organ while the device is deployed therein and attached to the tissue.

While typically the device has a single compartment, it is also envisaged that the device can comprise two or more compartments, similar or different, as shown, for example, in Figs. 3C-3D, which can be arranged to form a hollow lumen therebetween to permit passage of fluids and solids through the organ after expansion of the device (thus preventing blockage of the organ).

Figs 3E-3H show examples of cross sections of the device in a direction perpendicular to those show in Figs. 3A-3D. Figs. 3E and 3F correspond to Figs. 3A and 3B, respectively. Figs. 3F and 3G show cross sections where the device is designed to conform to a lumen or cavity with a varying cross section. The device may also be designed to conform to a cavity having a non-cylindrical form.

Fig. 3I shows a cross section of a device, in the same direction as that of Figs. 3E-3H, comprising of compartments with different lengths.

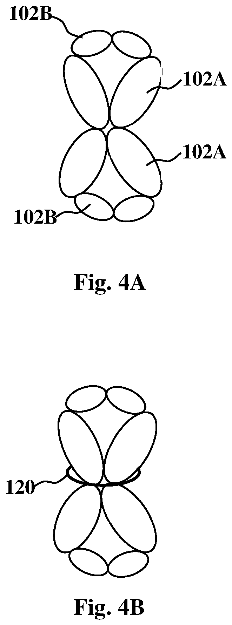

As shown in Figs. 4A-4B, the device can have other expanded shapes. For example, the device can comprise compartments having different sizes and configurations, resulting in three-dimensional (3D) shapes having different mechanical strengths. Further, some of the compartments can be attached to one another in one or more attachment locations, as to constrain the expansion of the device to form a desired 3D configuration. For example, while the devices shown in Figs. 3C-3D, each compartment is attached to two adjacent compartments to form a hollow closed shape when in the expanded state - in the device of Fig. 4A some of the compartments **102B** are attached to two adjacent compartments, while some of the compartments **102A** are attached to more than two adjacent compartments, thereby forcing the expanded device to assume an hourglass shape. Such a shape can provide increased rigidity and stability of the device. in such a configuration, not all of the compartments typically carry a tissue-attachable layer, for example compartments **102A** may be utilized to provide mechanical properties to the device, while compartments **102B** can carry the tissue-attachable layer (not shown) and come into contact with the tissue.

Alternatively, the compartments can be constrained by one or more constraining elements **120,** forcing the compartments to assume a desired shape when in the expanded state, as seen in Fig. 4B.

Further, in order to obtain compactization of the device in its collapsed (non-expanded) state, the device may be folded in various folding configurations (i.e. primary folded configurations, not shown) and/or rolling configurations (*i.e.* secondary rolled configuration), to permit its unfolding (and/or unrolling) during transition from the collapsed state to the expanded state. In other words, the device may be folded to assume its collapsed state, having an overall reduced size or overall reduced volume. Once liquid permeates through the deformable film, the gel-forming material starts to swell and increase in volume. This, in turn, applies force onto the deformable film, and due to its flexibility and/or deformability, the film is unfolded to assume the device's expanded state.

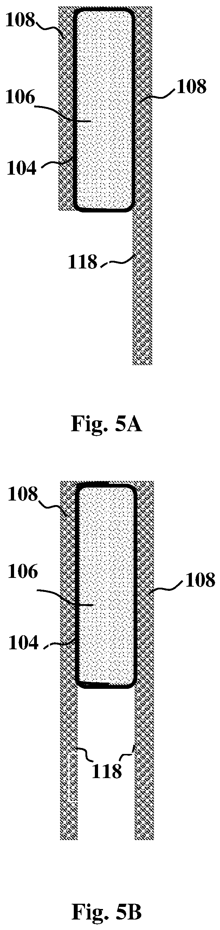

In some additional embodiments, as shown in Figs. 5A-5B, the tissue-attachable layer extends beyond the external surface of the compartment. In such embodiments, typically, an additional backing layer **118** is used, to provide support to the tissue-attachable layer.

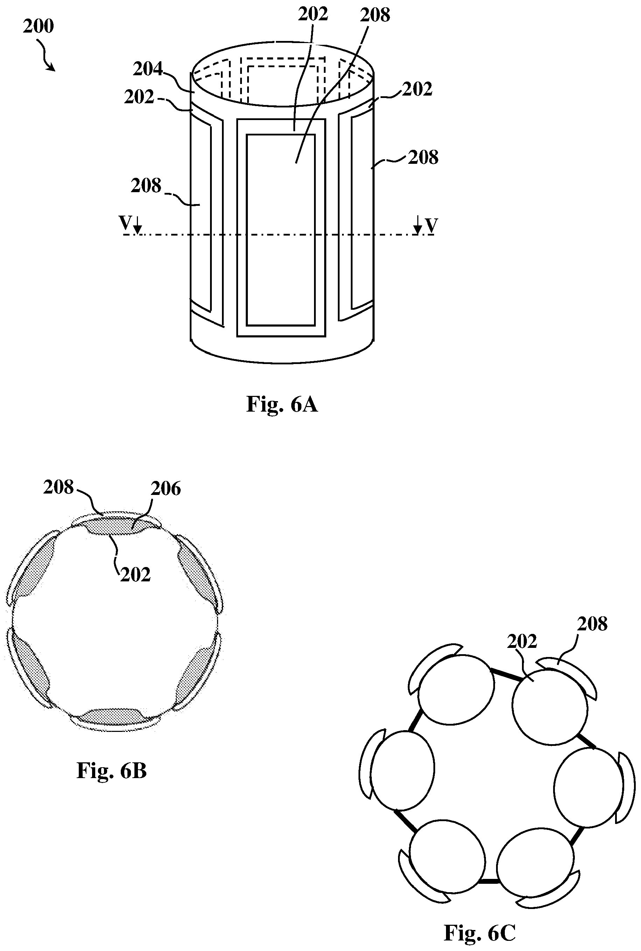

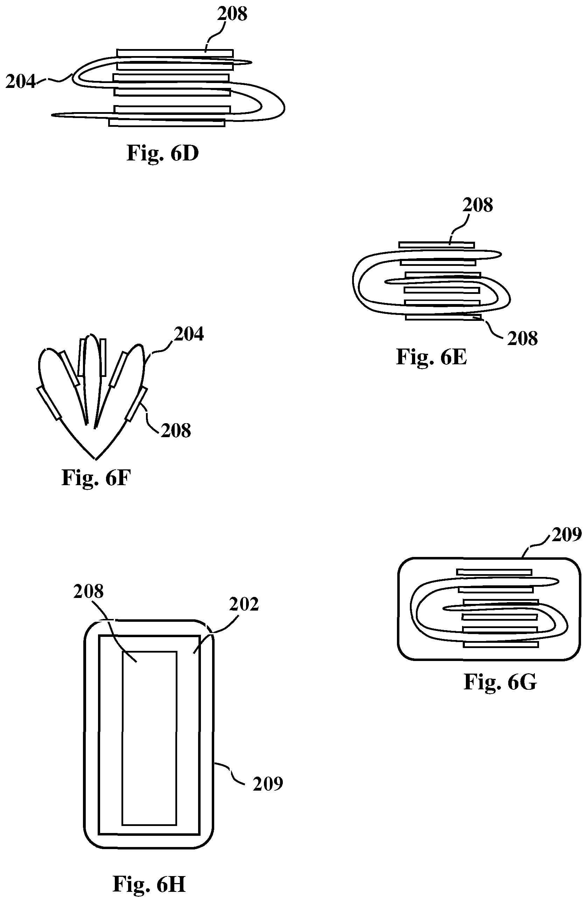

By some embodiments, similarly to the devices in Figs. 3C-3D, the device can assume a sleeve-like form, as shown in Figs. 6A-7B. The device **200** of Figs. 6A-6B has an overall cylindrical sleeve-like shape, and comprises a plurality, in this case six, self-expandable compartments, collectively designated **202,** and are formed out of a continuous deformable film **204.** While in this example six compartments are shown, it is to be understood that any number of compartments can be utilized, *e.g.* 2, 3, 4, 5, 6, 7, 8 or even more compartments.

In this example, the entire sleeve constitutes the deformable film, in which the compartments **202** are defined (as pockets made of liquid-permeable material and each enclosing a gel-forming material **206.** A tissue-attachable layer **208** is located on the external surface of each compartment **202** (*i.e.* the face of the film **204** that faces outwards from the compartment). The tissue-attachable layer **208,** as noted above, can comprise one or more active agents(s) to be delivered to a tissue.

The tissue-attachable layer **208** can be provided as patches that are applied (*e.g.* adhered) onto film **204** over compartments **202.** For example, when the tissue-attachable layer is a mucoadhesive layer, the patch can comprise the mucoadhesive material, layered upon a backing layer (not shown) that enables adhering or attaching the patch onto the external surface of the compartment and provides support for the mucoadhesive material. Alternatively, the mucoadhesive layer can be first applied at specific locations onto film **204,** followed by formation of the compartments **202** at locations corresponding to areas of the film containing the mucoadhesive layer, thus forming a multilayer film structure.

Seen in Fig. 6C is the device at its expanded form, *i.e.* after being exposed to liquids that cause swelling of the gel-forming material, thereby expanding the device.

Figs. 6D-6F show various configurations for folding the device of Figs. 6A-6C, in order to render it with a more compact form for intake. For ease of visualization, only tissue-attachable layers **208** are shown onto the film **204.** When in the collapsed state shown in Fig. 6A, the device can be folded to one of the primary folded configurations shown in Figs. 6D-6F, and encased in a biodegradable capsule (not shown). After intake and disintegration of the biodegradable capsule, exposure to liquid in the GI track causes expansion of the gel-forming material in the compartment, thereby expanding the compartment and causing at least partial concomitant expansion and unfolding into the expanded state.

Once folded, into the primary folded configuration, the folded device can be enveloped by an enteric envelope **209,** as shown for example in Figs. 6G-6H, functioning to maintain the device in its primary folded configuration until reaching proper conditions for deployment within the GI tract.

In order to obtain further compactization in the collapsed state, the device, by some embodiments, may have has a secondary, rolled configuration (not shown), whereby the folded device is further rolled about an axis thereof, and is configured to simultaneously undergo unrolling and unfolding during transition from the collapsed state to the expanded state. It is noted that an enteric envelope can envelope the device in its secondary folded configuration (not shown), instead or in addition to the enteric envelope **209** enveloping the device in its primary folded configuration. In cases where the device includes two enteric envelopes, the first and second envelopes can be configured to have the same disintegration/dissolution properties or different disintegration/dissolution properties.

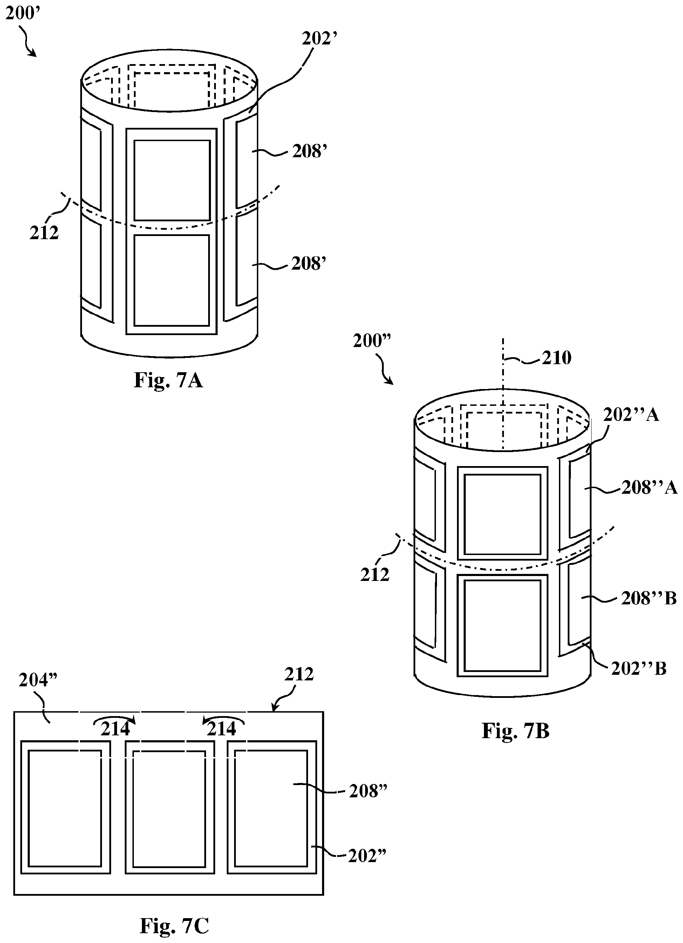

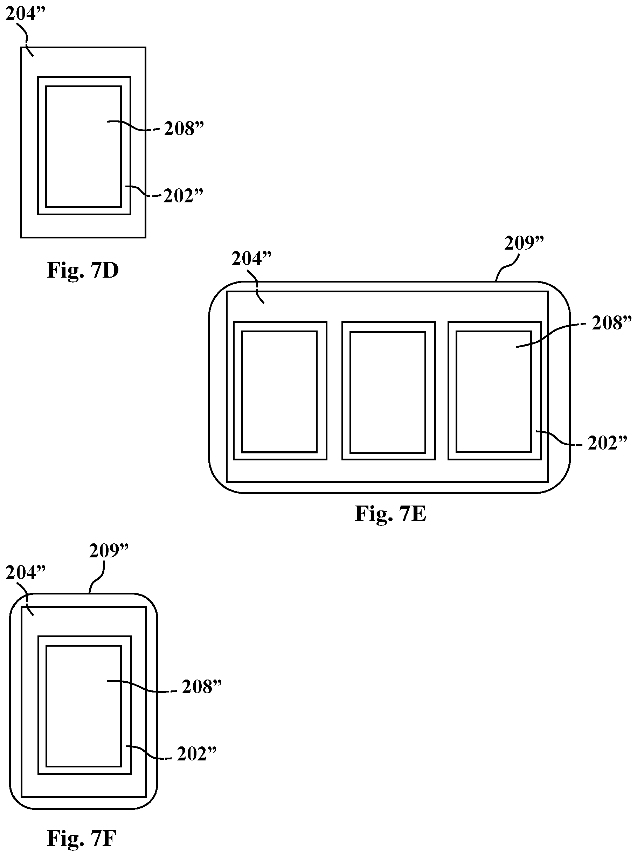

Figs. 7A-7B show alternative arrangements of the device of Figs. 6A-6B. In the device **200'** shown in Fig. 7A, each compartment **202'** includes two regions coated by a layer **208'** of mucoadhesive material. It is noted that while in the examples of Figs. 7A-7B two regions are shown, a person of skill in the art would appreciate that more than two regions can be utilized over each compartment, *e.g.* 3, 4, 5, 6 or even more such regions, and the devices described herein are not limited by the number of such regions.

Alternatively, as seen in Fig. 7B, the device **200"** can include pairs of compartments **202"A,202"B,** the compartments in each pair being arranged consecutively along the longitudinal axis **210** of the device, and each compartment **202A,202B** carrying a mucoadhesive layer **208"** on its external surface. The arrangements shown in Figs. 7A-7B permit additional folding of the device along line **212,** thereby permitting the device to assume a further compacted form suitable for intake. For example, as shown Fig. 7C, the device of Fig. 7B can be flattened and then folded over line **212** to obtain a folded collapsed device. The device of Fig. 7C can be further folded, *e.g.* in the direction of arrows **214,** to further reduce the size of the device and obtain the primary folded configuration shown in Fig. 7D. As can be seen in Figs. 7E-7F, the devices in their primary folded configurations can be enveloped by at least one enteric envelope **209"**.

The devices can also comprise at least one additional active substance, being different from the active agent. The additional active substance can have similar pharmaceutical activity as the active agent, or have a different pharmaceutical activity to that of the active agent. For example, the additional active substance can be contained in the mucoadhesive layer, to be released concomitantly or sequentially with the active agent. By another example, the additional active substance can function to increase permeation or bioavailability of the active agent, or can increase or enhance the therapeutic effect or bioactivity of the active agent.

By a further example, the additional active substance can be contained within the compartment, *e.g.* associated with the deformable film, associated with the gel-forming material, or mixed (or dispersed) into the gel-forming material. In such cases, the additional active substance may be selected to have an immediate or a short-term therapeutic effect, while the active agent can be selected to have a prolonged or sustained therapeutic effect.

**Claims**

1. A self-expandable device (100, 200, 200', 200") configured for attaching a tissue-attachable layer to a tissue, the device having a collapsed state and an expanded state, and comprising:

   at least one self-expandable compartment (102, 202, 202', 202") formed out of a substantially continuous, deformable film (104, 204) having one or more liquid-permeable sections, the compartment enclosing a gel forming material therein, the gel forming material (106, 206) being configured for swelling upon contact with liquid, thereby expanding the compartment to irreversibly switch the device from the collapsed state to the expanded state; and

   a tissue-attachable layer (108, 208, 208', 208") coating at least a portion of an external surface of the compartment, and comprises at least one active agent to be delivered to the tissue,

   such that expansion of the device from the collapsed state to the expanded state causes expansion of the compartment to drive the tissue-attachable layer towards said tissue for attaching at least part of the tissue-attachable layer to the tissue.

2. The device of claim 1, wherein the tissue-attachable layer comprises a plurality of microneedles.

3. The device of claim 1, wherein the tissue-attachable layer is a mucoadhesive layer.

4. The device of claim 3, wherein the mucoadhesive layer comprises at least one mucoadhesive material.

5. The device of claim 3, wherein the mucoadhesive layer comprises at least one mucoadhesive material and said at least one active agent.

6. The device of claim 2, wherein the microneedles are comprised of a polymeric material in which said at least one active agent is embedded.

7. The device of claim 5 or 6, wherein the active agent is a pharmaceutical active agent.

8. The device of any one of claims 1 to 7, wherein said one or more sections of the deformable film differ one from the other in their liquid permeability.

9. The device of any one of claims 1 to 8, wherein the deformable film is disintegrable.

10. The device of any one of claims 1 to 9, wherein the deformable film comprises weak regions permitting fragmentation of the film.

11. The device of any one of claims 1 to 10, wherein the gel forming material is disintegrable.

12. The device of any one of claims 1 to 11, wherein, when in the collapsed state, the device is folded in a primary folded configuration and is configured to undergo unfolding during transition from the collapsed state to the expanded state, optionally wherein when in the collapsed state, the device has a secondary, rolled configuration, whereby the folded device is further rolled about an axis thereof, and is configured to simultaneously undergo unrolling and unfolding during transition from the collapsed state to the expanded state.

13. The device of any one of claims 1 to 12, wherein, when in the collapsed state, the device is rolled about an axis thereof, and is configured to undergo unrolling during transition from the collapsed state to the expanded state.

14. The device of any one of claims 1 to 13, comprising a biodegradable shell (110, 116, 209), encapsulating the device in its collapsed state.

15. The device of any one of claims 1 to 14, being an ingestible device.

16. A self-expandable device of any one of claims 1 to 15 for use in attaching, through a mucous membrane, a tissue-attachable layer (108, 208, 208', 208") to a tissue of a subject in need thereof, the self-expandable device (100, 200, 200', 200") is encapsulated in a biodegradable shell (110, 116, 209).

17. A self-expandable device (100, 200, 200', 200") of any one of claims 1 to 15 for use in delivery of at least one active agent to a tissue of a subject in need thereof, the self-expandable device is encapsulated in a biodegradable shell (110, 116, 209).

**Patentansprüche**

1. Selbstexpandierbare Vorrichtung (100, 200, 200', 200"), welche dazu eingerichtet ist, eine gewebeanbringbare Schicht an einem Gewebe anzubringen, wobei die Vorrichtung einen kollabierten Zustand und einen expandierten Zustand aufweist, und umfassend:

   wenigstens eine selbstexpandierbare Kammer (102, 202, 202', 202", welche aus einer im Wesentlichen kontinuierlichen, verformbaren Folie (104, 204) gebildet ist, die einen oder mehrere flüssigkeitsdurchlässige Abschnitte aufweist, wobei die Kammer ein gelbildendes Material darin einschließt, wobei das gelbildende Material (106, 206) dazu eingerichtet ist, bei einem Kontakt mit Flüssigkeit aufzuquellen, wodurch die Kammer expandiert wird, um die Vorrichtung irreversibel von dem kollabierten Zustand in den expandierten Zustand zu überführen; und
   eine gewebeanbringbare Schicht (108, 208, 208', 208"), welche wenigstens einen Teil einer Außenfläche der Kammer bedeckt und wenigstens einen an das Gewebe abzugebenden Wirkstoff umfasst,
   so dass eine Expansion der Vorrichtung von dem kollabierten Zustand in den expandierten Zustand eine Expansion der Kammer bewirkt, um die gewebeanbringbare Schicht in Richtung des Gewebes zu treiben, um wenigstens einen Teil der gewebeanbringbaren Schicht an dem Gewebe anzubringen.

2. Vorrichtung nach Anspruch 1, wobei die gewebeanbringbare Schicht eine Mehrzahl von Mikronadeln umfasst.

3. Vorrichtung nach Anspruch 1, wobei die gewebeanbringbare Schicht eine mukoadhäsive Schicht ist.

4. Vorrichtung nach Anspruch 3, wobei die mukoadhäsive Schicht wenigstens ein mukoadhäsives Material umfasst.

5. Vorrichtung nach Anspruch 3, wobei die mukoadhäsive Schicht wenigstens ein mukoadhäsives Material und den wenigstens einen Wirkstoff umfasst.

6. Vorrichtung nach Anspruch 2, wobei die Mikronadeln aus einem polymeren Material bestehen, in welches der wenigstens eine Wirkstoff eingebettet ist.

7. Vorrichtung nach Anspruch 5 oder 6, wobei der Wirkstoff ein pharmazeutischer Wirkstoff ist.

8. Vorrichtung nach einem der Ansprüche 1 bis 7, wobei sich der eine oder die mehreren Abschnitte der verformbaren Folie in ihrer Flüssigkeitsdurchlässigkeit voneinander unterscheiden.

9. Vorrichtung nach einem der Ansprüche 1 bis 8, wobei die verformbare Folie zersetzbar ist.

10. Vorrichtung nach einem der Ansprüche 1 bis 9, wobei die verformbare Folie Schwachstellenbereiche umfasst, welche eine Fragmentierung der Folie ermöglichen.

11. Vorrichtung nach einem der Ansprüche 1 bis 10, wobei das gelbildende Material zersetzbar ist.

12. Vorrichtung nach einem der Ansprüche 1 bis 11, wobei, wenn sie sich in dem kollabierten Zustand befindet, die Vorrichtung in eine primär gefaltete Konfiguration gefaltet ist und dazu eingerichtet ist, während eines Übergangs von dem kollabierten Zustand in den expandierten Zustand ein Entfalten zu erfahren, optional wobei, wenn sie sich in dem kollabierten Zustand befindet, die Vorrichtung eine sekundäre, gerollte Konfiguration aufweist, wobei die gefaltete Vorrichtung ferner um eine Achse davon gerollt ist, und dazu eingerichtet ist, während eines Übergangs von dem kollabierten Zustand in den expandierten Zustand gleichzeitig ein Entrollen und ein Entfalten zu erfahren.

13. Vorrichtung nach einem der Ansprüche 1 bis 12, wobei, wenn sie sich in dem kollabierten Zustand befindet, die Vorrichtung um eine Achse davon gerollt ist und dazu eingerichtet ist, während eines Übergangs von dem kollabierten Zustand in den expandierten Zustand ein Entrollen zu erfahren.

14. Vorrichtung nach einem der Ansprüche 1 bis 13, umfassend eine biologisch abbaubare Hülle (110, 116, 209), welche die Vorrichtung in ihrem kollabierten Zustand einkapselt.

15. Vorrichtung nach einem der Ansprüche 1 bis 14, wobei es sich um eine verzehrbare Vorrichtung handelt.

16. Selbstexpandierbare Vorrichtung nach einem der Ansprüche 1 bis 15 zur Verwendung bei einem Anbringen, durch eine Mukosalmembran, einer gewebeanbringbaren Schicht (108, 208, 208', 200") an einem Gewebe eines behandlungsbedürftigen Subjekts, wobei die selbstexpandierbare Vorrichtung (100, 200, 200', 200") in einer biologisch abbaubaren Hülle (110, 116, 209) eingekapselt ist.

17. Selbstexpandierbare Vorrichtung (100, 200, 200', 200") nach einem der Ansprüche 1 bis 15 zur Verwendung bei einer Abgabe wenigstens eines Wirkstoffs an ein Gewebe eines behandlungsbedürftigen Subjekts, wobei die selbstexpandierbare Vorrichtung in einer biologisch abbaubaren Hülle (110, 116, 209) eingekapselt ist.

**Revendications**

1. Dispositif auto-dilatable (100, 200, 200', 200") configuré pour fixer une couche fixable au tissu à un tissu, le dispositif ayant un état affaissé et un état dilaté, et comprenant :

   au moins un compartiment auto-dilatable (102, 202, 202', 202") formé d'un film déformable sensiblement continu (104, 204) présentant une ou plusieurs sections perméables aux liquides, le compartiment enserrant un matériau gélifiant en son sein, le matériau gélifiant (106, 206) étant configuré pour gonfler au contact d'un liquide, dilatant ainsi le compartiment pour commuter irréversiblement le dispositif de l'état affaissé à l'état dilaté ; et
   une couche fixable au tissu (108, 208, 208', 208") revêtant au moins une portion d'une surface externe du compartiment, et comprenant au moins un agent actif à délivrer au tissu,
   de sorte qu'une dilatation du dispositif de l'état affaissé à l'état dilaté provoque une dilatation du compartiment pour entraîner la couche fixable au tissu vers ledit tissu pour fixer au tissu au moins une partie de la couche fixable au tissu.

2. Dispositif selon la revendication 1, dans lequel la couche fixable au tissu comprend une pluralité de microaiguilles.

3. Dispositif selon la revendication 1, dans lequel la couche fixable au tissu est une couche mucoadhésive.

4. Dispositif selon la revendication 3, dans lequel la couche mucoadhésive comprend au moins un matériau mucoadhésif.

5. Dispositif selon la revendication 3, dans lequel la couche mucoadhésive comprend au moins un matériau mucoadhésif et ledit au moins un agent actif.

6. Dispositif selon la revendication 2, dans lequel les microaiguilles sont constituées d'un matériau polymérique dans lequel ledit au moins un agent actif est intégré.

7.  Dispositif selon la revendication 5 ou 6, dans lequel l'agent actif est un agent actif pharmaceutique.

8.  Dispositif selon l'une quelconque des revendications 1 à 7, dans lequel lesdites une ou plusieurs sections du film déformable diffèrent les unes des autres par leur perméabilité aux liquides.

9.  Dispositif selon l'une quelconque des revendications 1 à 8, dans lequel le film déformable est désintégrable.

10. Dispositif selon l'une quelconque des revendications 1 à 9, dans lequel le film déformable comprend des régions fragiles permettant la fragmentation du film.

11. Dispositif selon l'une quelconque des revendications 1 à 10, dans lequel le matériau gélifiant est désintégrable.

12. Dispositif selon l'une quelconque des revendications 1 à 11, dans lequel, lorsqu'il est à l'état affaissé, le dispositif est plié en une configuration pliée primaire et est configuré pour subir un dépliage pendant une transition de l'état affaissé à l'état dilaté, facultativement dans lequel, lorsqu'il est à l'état affaissé, le dispositif présente une configuration enroulée secondaire, moyennant quoi le dispositif plié est en outre enroulé autour d'un axe de celui-ci, et est configuré pour subir simultanément un déroulement et un dépliage pendant une transition de l'état affaissé à l'état dilaté.

13. Dispositif selon l'une quelconque des revendications 1 à 12, dans lequel, lorsqu'il est à l'état affaissé, le dispositif est enroulé autour d'un axe de celui-ci, et est configuré pour subir un déroulement pendant une transition de l'état affaissé à l'état dilaté.

14. Dispositif selon l'une quelconque des revendications 1 à 13, comprenant une coque biodégradable (110, 116, 209), encapsulant le dispositif dans son état affaissé.

15. Dispositif selon l'une quelconque des revendications 1 à 14, qui est un dispositif ingérable.

16. Dispositif auto-dilatable selon l'une quelconque des revendications 1 à 15 pour une utilisation dans la fixation, à travers une membrane muqueuse, d'une couche fixable au tissu (108, 208, 208', 208") à un tissu d'un sujet qui en a besoin, le dispositif auto-dilatable (100, 200, 200', 200'') est encapsulé dans une coque biodégradable (110, 116, 209).

17. Dispositif auto-dilatable (100, 200, 200', 200") selon l'une quelconque des revendications 1 à 15 pour utilisation dans la délivrance d'au moins un agent actif à un tissu d'un sujet qui en a besoin, le dispositif auto-dilatable est encapsulé dans une coque biodégradable (110, 116, 209).

Fig. 1A

Fig. 1B

Fig. 1C

Fig. 1D

Fig. 1E

110

111

111

116

100

**Fig. 1F**

113   110   115   113   111

111

116

100

**Fig. 1G**

Fig. 2A

Fig. 2B

Fig. 2C

Fig. 2D

Fig. 2E

Fig. 2F

Fig. 2G

Fig. 2H

Fig. 3A

Fig. 3B

Fig. 3C

Fig. 3D

Fig. 3E    Fig. 3F    Fig. 3G    Fig. 3H    Fig. 3I

Fig. 4A

Fig. 4B

108

106

104

108

118

**Fig. 5A**

108

106

104

108

118

**Fig. 5B**

**Fig. 6A**

**Fig. 6B**

**Fig. 6C**

**Fig. 6D**

**Fig. 6E**

**Fig. 6F**

**Fig. 6G**

**Fig. 6H**

200'

202'

208'

212

208'

**Fig. 7A**

200"

210

202"A

208"A

212

208"B

208"B

202"B

**Fig. 7B**

204"

212

214

214

208"

202"

**Fig. 7C**

204"

208"

202"

**Fig. 7D**

209"

204"

208"

202"

**Fig. 7E**

209"

204"

208"

202"

**Fig. 7F**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2016015648 A
- WO 2008062440 A
- WO 2009125432 A
- WO 2013188819 A
- WO 2015026552 A
- WO 2015120471 A